Europäisches Patentamt

European Patent Office

Office européen des brevets

(11) Veröffentlichungsnummer: **0 264 047 B1**

## (12) EUROPÄISCHE PATENTSCHRIFT

(45) Veröffentlichungstag der Patentschrift: **19.01.94**

(21) Anmeldenummer: **87114527.2**

(22) Anmeldetag: **05.10.87**

(51) Int. Cl.⁵: **G01L 1/14**, G01D 5/24

(54) Kapazitiver Messanordnung zur Bestimmung von Kräften und/oder Drücken.

(30) Priorität: **13.10.86 DE 3634855**

(43) Veröffentlichungstag der Anmeldung:
**20.04.88 Patentblatt 88/16**

(45) Bekanntmachung des Hinweises auf die
Patenterteilung:
**19.01.94 Patentblatt 94/03**

(84) Benannte Vertragsstaaten:
**DE FR GB IT**

(56) Entgegenhaltungen:
**EP-A- 0 172 784**
**DE-A- 2 529 475**
**DE-A- 3 411 528**
**FR-A- 2 513 508**
**GB-A- 1 321 831**

(73) Patentinhaber: **Seitz, Peter**
**Möhlstrasse 29**
**D-81675 München(DE)**

(72) Erfinder: **Seitz, Peter**
**Möhlstrasse 29**
**D-81675 München(DE)**

(74) Vertreter: **Bohnenberger, Johannes, Dr. et al**
**Meissner, Bolte & Partner**
**Postfach 86 06 24**
**D-81633 München (DE)**

**Beschreibung**

Die Erfindung betrifft eine kapazitive Meßanordnung nach dem Oberbegriff des Hauptanspruches.

In vielen Bereichen der Technik, des Sportes oder der Medizin ist es nötig, zur Untersuchung bestimmter Phänomene die bei einem dynamischen Vorgang auftretenden Kräfte nicht nur ihrem Summenwert nach, sondern auch hinsichtlich ihrer Verteilung zu bestimmen. So zum Beispiel ist es für den Hersteller eines Autositzes äußerst wünschenswert zu wissen, wie die tatsächliche Druckverteilung auf der Sitzfläche und der Rückenlehne aussieht, wenn eine Person darauf sitzt, damit der Sitz an den höher belasteten Stellen haltbarer bzw. steifer ausgestaltet werden kann als an niedriger belasteten Stellen. Bei der Herstellung eines Skis ist es wünschenswert, die Kraftverteilung zu kennen, damit die Kernkonstruktion dieser Verteilung entsprechend angepaßt werden kann. In der Medizin schließlich kann man aus dem Kraft-Verteilungsmuster unter den Fußflächen beim Gehen eines Probanden auf orthopädische Schäden oder auch auf sensorische Schädigungen (z.B. bei Zuckerkranken) schließen und die entsprechenden Therapiemaßnahmen einleiten.

Zur Messung der Kraftverteilung über Flächen ist aus der DE-A 25 29 475 eine Matrixanordnung von streifenförmigen Kondensatorelementen bekannt, die unter Zwischenschaltung eines elastischen Dielektrikums einander gegenüberliegend (und kreuzend) angeordnet sind. Bei dieser Anordnung führen lediglich Vertikalkräfte, also Kräfte senkrecht zu den Kondensatorflächen, zu Meßsignalen, da eine Horizontalverschiebung der streifenförmigen Kondensatorelemente keine Änderung der einander gegenüberliegenden Flächendimensionen bewirken. Nachteilig an der bekannten Anordnung ist der Umstand, daß die einzelnen Kondensatorelemente, die aus den einander direkt gegenüberliegenden Flächenabschnitten bestehen, mechanisch miteinander verbunden sind, so daß die Ortsauflösung begrenzt ist bzw. das gemessene Muster verfälscht wird.

Zur Entkopplung der einzelnen Kondensatorflächen wird in der DE-A 34 11 528 vorgeschlagen, die streifenförmigen Kondensatorflächen durch Schnitte in Einzelelemente aufzuteilen, wobei in dieser Druckschrift insbesondere die Anregung gegeben wird, zwischen den Einzelflächen mäanderförmige Stromleiterbahnen (durch die entsprechenden Schnitte) vorzusehen. Mit dieser Anordnung wird zwar eine sehr wirksame Entkopplung der Einzelflächen bewirkt, so daß die abzugreifenden Kraft-Verteilungsmuster wesentlich exakter als bisher sind, jedoch führen bei dieser Anordnung Kräfte parallel zu den Kondensatorflächen zu einer Veränderung der wirksamen Kondensatorflächen, also zu einer Verfälschung des Vertikal-Signales.

Beiden oben genannten Anordnungen ist schließlich gemeinsam, daß die in vielen Fällen wünschenswerte dreidimensionale Messung von Kraftverteilungen nicht möglich ist. Z.B. wäre die Kenntnis über die dreidimensionale Kraftverteilung für die (Auto-) Reifenindustrie sehr interessant, selbstverständlich ebenso für alle weiter oben beschriebenen Anwendungszwecke.

Aus der DE-A 34 10 955 ist eine kapazitive Meßanordnung zur Bestimmung von Kräften bekannt, bei der zwei Gruppen von zueinander parallelen Kondensatorplatten kammartig angeordnet ineinander greifend und parallel zueinander verschiebbar gehalten sind. Im Grundzustand, bei dem noch keine Kräfte auf die Kondensatorplatten bzw. deren Halterungen wirken, überdecken die Platten einander nicht vollständig, so daß bei Aufbringung von Relativkräften parallel zu den Oberflächen der Plattensätze diese weiter ineinander greifen, wodurch eine Erhöhung der Kapazität zwischen den Platten meßbar wird. Bei dieser Anordnung muß zur Vermeidung eines Meßfehlers sichergestellt sein, daß die Platten ausschließlich parallel zueinander verschoben werden, so daß auch bei Anwendung dieser bekannten Lehre keine dreidimensionale Kraftmessung oder eine Eliminierung von Kräften unzulässiger Richtung möglich ist.

Ausgehend vom oben genannten Stand der Technik ist es Aufgabe der vorliegenden Erfindung, eine Meßanordnung der eingangs genannten Art dahingehend weiterzubilden, daß neben der Kraftmessung in einer Richtung eine Eliminierung bzw. Messung einer Kraftkomponente in mindestens einer weiteren Richtung ermöglicht wird.

Diese Aufgabe wird durch die im Hauptanspruche angegebenen Merkmale gelöst. Man kann also gemäß der vorliegenden Erfindung entweder die durch die Scherwirkung im Dielektrikum wirkenden Kräfte messen und so eine mehrdimensionale Meßanordnung erhalten, oder aber das Meßergebnis, das ein Maß für die flächenparallel wirkenden Kräfte ist, verwerfen, um dafür die flächennormalen Kräfte exakter bestimmen zu können.

Bei einer bevorzugten Ausführungsform der Erfindung sind der Hauptfläche drei oder vier im wesentlichen kongruente, voneinander getrennte kleinere Flächen gegenüberliegend angeordnet, die mit ihren Außenrändern über den Außenrand der Hauptfläche hervorragen. Durch eine geeignete Verrechnung der Einzelmeßwerte lassen sich so Richtung und Amplitude des resultierenden (dreidimensional gesehenen) Kraftvektors bestimmen. Vorzugsweise sind hierbei die Hauptfläche und die Gegenflächen derart über die Kapazitätsmeßorgane und die Rechenschaltungen miteinander verschaltet, daß an den Ausgängen der Rechenschaltungen die Summe der Einzelkapazitäten - entsprechend der Vertikalkraft - und die Summen bzw. Differenzen der einzelnen Kapazitäten - entsprechend den Horizontalkräften - bezogen auf die Gesamtkapazität bzw. die Vertikalkraft am Ausgang der Rechenschaltung anstehen. Die Normalisierung der Horizontalkräfte (flächenparallele Kräfte) durch die Vertikalkraft ist notwendig, weil bei der Vertikalkraftmessung die Kondensatorfläche konstant bleibt, während der Abstand zwischen den Kondensatorplatten variiert, beim Wirken von Horizontalkräften, also flächenparallelen Kräften aber die Flächen variieren, während der Abstand zwischen den Kondensatorelemenen - wie vorher beschrieben - ebenfalls variieren kann.

Vorzugsweise ist eine Vielzahl von Hauptflächen und Gegenflächen vorgesehen, die gleichartig ausgebildet und äquidistant mit jeweils korrespondieren Haupt- bzw. Gegenflächen in einer Matrixanordnung angeordnet sind. Mit einer derartigen Meßanordnung läßt sich eine dreidimensionale Kraftverteilung über eine größere Fläche hinweg messen, wobei die einzelnen Meßelemente bzw. Meßpunkte voneinander vollständlich mechanisch entkoppelbar sind.

Die Anordnung wird dann besonders leicht, dünn und einfach herzustellen, wenn die Kondensatorflächen als dünne Schichten ausgebildet und auf Kunststoffolien aufgebracht (aufgedruckt) sind, die unter Zwischenschaltung des elastomeren Dielektrikums miteinander verbunden (verklebt) sind, wobei die Kunststoffolien zwischen den Kondensatorflächen durch Schnitte oder Kerben durchtrennt sind. Die so entstehenden Matrixanordnungen können äußerst kostengünstig gefertigt werden und zwar auch als relativ dünne "Folien", so daß sie sich z.B. in Schuhen als Einlegesohlen anbringen lassen. Durch die Schnitte werden die einzelnen Sensorelemente voneinander entkoppelt, wobei die Schnitte dann, wenn auf beiden Außenflächen noch zusätzlich Abschirmflächen angebracht sind, auch diese mit durchtrennen. Die Schnitte führen mindestens bis an das elastomere Dielektrikum bzw. gegebenenfalls auch noch ein Stück in dieses hinein.

Besonders hoch ist die Empfindlichkeit dann, wenn das Dielektrikum ein geschäumtes, vorzugsweise geschlossenporiges Elastometer umfaßt. Vorzugsweise besteht aber das Dielektriukum aus zwei Schichten, die einander gegenläufige Temperaturkoeffizienten - hinsichtlich ihrer Dielektrizitätskonstante - haben, wobei die mechanische Dimensionierung so getroffen ist, daß ein gemeinsamer Temperaturkoeffizient von im wesentlichen Null erhalten wird. Auf diese Weise gelingt es, die Temperatureinflüsse auf die einzelnen Sensorkapazitäten zu eliminieren, so daß eine einmal vorgenommene Eichung der Sensoren bzw. eines Einzelsensors über eine längere Versuchsdauer hinweg, trotz schwankender Temperaturen, ihre Gültigkeit behält.

Nachfolgendwenden Ausführungsbeispiele, anhand von Abbildungen näher erläutert. Hierbei zeigen:

Fig. 1    eine Anordnung mit drei Gegenflächen;

Fig. 2    eine Anordnung mit vier Gegenflächen;

Fig. 3    eine Matrixanordnung mit jeweils vier Gegenflächen;

Fig. 4    einen Schnitt entlang der Linie V-V aus Fig. 3;

Fig. 5    eine Prinzip-Schaltung zur dreidimensionalen Kraftmessung;

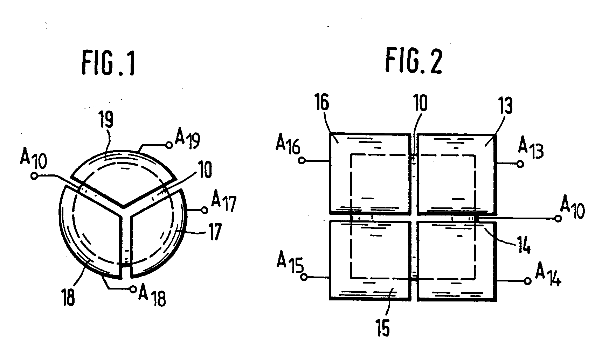

Bei den in den Fig. 1 und 2 gezeigten Anordnungen können die Kräfte in x- oder y-Richtung gemessen werden. Bei diesen Anordnungen liegt (Fig. 1) einer kreisförmigen kleineren Kondensatorfläche 10 ein Satz (3) von Sektor-Kondensatorflächen 17 bis 19 gegenüber, die voneinander elektrisch getrennt sind. Die Sektorflächen 12 bis 19 weisen einen größeren Außenumfang auf als die Kondensatorfläche 10. Tritt nun bei dieser Meßanordnung eine Kraft in x- oder in y-Richtung auf, so verschiebt sich die Kondensatorfläche 10 parallel zu den Sektorflächen 17 bis 19, so daß sich die Einzelkapazitäten ändern. Besonders übersichtlich wird die Veränderung dann, wenn man insgesamt vier (quadratische) Gegenflächen 13 bis 16 gegenüber einer kleineren Kondensatorfläche 10 anordnet, wie dies in Fig. 2 gezeigt ist. Wirkt in diesem Fall eine Kraft in x-Richtung (in Fig. 2 von links nach rechts) auf die Kondensatorfläche 10, so daß diese relativ zu den Gegenflächen verschoben wird, so sinken die Kapazitäten zwischen den Kondensatorflächen 15 und 16 und der Fläche 10, während die Kapazitäten zwischen den Kondensatorflächen 13 und 14 und der Fläche 10 steigen. Aus der Differenz läßt sich dann die Größe der Kraft dem Betrag nach herleiten.

Wirkt gleichzeitig noch eine Kraft senkrecht zu den Kondensatorflächen 10 bis 16, also in z-Richtung, so muß der Einfluß dieser Kraft auf die Einzelkapazitäten aus dem Meßergebnis für die Kräfte in x- und y-Richtung eliminiert werden. Dies geschieht dadurch, daß man eine der Kraft z bzw. dem Abstand der Flächen 13 bis 16 zur Fläche 10 proportionale Größe als Normalisierungsfaktor in die Differenzmessung mit einfließen läßt.

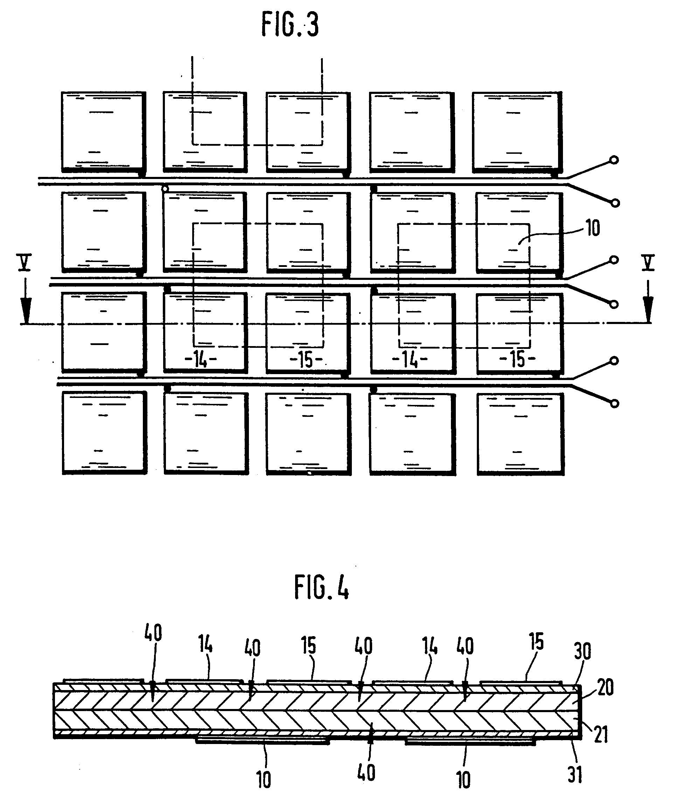

In Fig. 3 ist der Ausschnitt einer Matrixanordnung, bestehend aus Elementen gemäß Fig. 2 schematisiert dargestellt. Aus der Abbildung ist hierbei ersichtlich, daß immer gleiche (gleiche Signale erzeugende) Kondensatorflächen elektrisch zusammengefaßt und mit einem gemeinsamen Außenanschluß verbunden sind. Es liegen z.B. alle Kondensatorflächen 14 einer Zeile auf derselben Leitung. Selbstverständlich sind die Kondensatorflächen 10 zu Spalten elektrisch zusammengefaßt, so daß sich bei dieser Matrixanordnung jedes Sensorelement, bestehend aus jeweils vier Einzelkondensatoren, gesondert "auslesen" läßt.

Aus der in Fig. 4 gezeigten Schnittdarstellung entlang der Linie V-V aus Fig. 3 geht eine weitere Besonderheit des hier gezeigten Ausführungsbeispieles hervor. Dabei ist zu erwähnen, daß die in Fig. 4 gezeigte Darstellung in keiner Weise maßstäblich ist.

Bei dieser Ausführungsform sind die Kondensatorflächen 10, 14 und 15 auf Folien 30 bzw. 31 aufgedruckt. Die Folien 30 und 31 sind dann jeweils auf ein kompressibles Dielektrikum (Elastomer) 20 bzw. 21 aufgeklebt, wobei diese beiden Dielektrika 20, 21 aufeinander liegend verbunden sind. Die Dielektrika 20 und 21 weisen gegenläufige Temperaturkoeffizienten hinsichtlich ihrer dielektrischen Eigenschaften auf und sind so bemessen, daß die Temperatur keinen Einfluß auf die Kapazität der Einzel-Kondensatoren hat.

Die Folien 30 und 31 sind durch Kerben 40 zwischen den Kondensatorflächen 14 und 15 bzw. 10 durchtrennt, so daß keinerlei mechanische Kopplung über die Folien 30 und 31 zwischen den Einzelflächen auftreten kann. Weiterhin wird durch die Anbringung der Schnitte oder Kerben 40 die Flexibilität der Gesamtanordnung wesentlich erhöht.

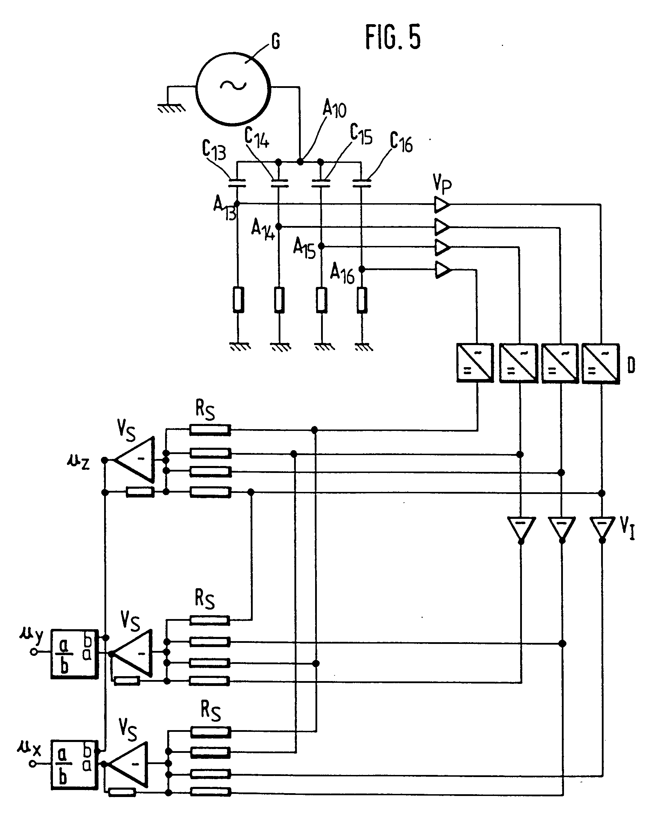

In Fig. 5 ist eine Prinzip-Schaltung zur Auswertung der Meßsignale gezeigt. Hierbei bringt ein Hochfrequenzgenerator G ein Wechselstromsignal (gegenüber Masse) auf die Hauptfläche 10, während die Gegenflächen 13 bis 16 über Widerstände gegen Masse geschaltet sind. Somit entstehen vier Spannungsteiler, bestehend aus den Einzelkapazitäten $C_{13}$ bis $C_{16}$ und den auf Masse führenden Widerständen. Die heruntergeteilten Spannungen werden über Pufferverstärker $V_P$ Demodulatoren D zugeleitet, welche die Wechselstromsignale in Gleichstromsignale wandeln.

An den Ausgängen der Demodulatoren D werden die Meßspannungen über Summierwiderstände $R_S$ und einen gegengekoppelten Summierverstärker $V_S$ summiert, so daß sich ein Ausgangssignal $U_z$ ergibt, das der Summenkapazität proportional ist. Weiterhin werden die Ausgänge der Demodulatoren G über Invertierverstärker $V_I$ invertiert.

Die den Kapazitäten $C_{13}$ und $C_{14}$ entsprechenden Werte werden weiterhin mit den invertierten Werten der Kapazitäten $C_{15}$ und $C_{16}$ über einen weiteren Summierverstärker $V_S$ und Summierwiderstände $R_S$ miteinander verrechnet. Das Summenbzw. Differenzsignal wird dann in einer Dividierschaltung durch das Summensignal $U_z$ dividiert, so daß ein Signal $U_y$ entsteht, welches der in y-Richtung an der kapazitiven Meßanordnung nach Fig. 2 wirkenden Kraft proportional ist, wobei die z-Komponente durch die Division eliminiert ist. In analoger Weise wird das der x-Richtung entsprechende Signal $U_x$ erzeugt.

Selbstverständlich ist nicht nur die in Fig. 5 gezeigte Analogschaltung möglich, vielmehr wird die Verrechnung (nach Multiplexen der Ausgangssignale $A_{13}$ bis $A_{16}$) digital durchgeführt.

**Patentansprüche**

1. Kapazitive Meßanordnung zur Bestimmung von Kräften und/oder Drücken mit Kondensatorflächen (10; 13 - 19), die in zwei Ebenen mit dazwischenliegendem Dielektrikum (20, 21) angeordnet sind und einander im wesentlichen gegenüber liegen, wobei die Kondensatorflächen (10) in der einen Ebene relativ zu den Kondensatorflächen (13 - 19) in der anderen Ebene entgegen elastischen Rückelementen beweglich gehalten sind, **dadurch gekennzeichnet,** daß die Kondensatorflächen eine Hauptfläche (10) umfassen, die in der einen Ebene angeordnet ist, während alle übrigen Kondensatorflächen als Gegenflächen (13 - 19) zur Hauptfläche (10) in der anderen Ebene unter Zwischenschaltung des Dielektrikums (20, 21) angeordnet sind, wobei die Hauptfläche (10) sowohl senkrecht als auch parallel relativ zu den Gegenflächen (13 - 19) bewegbar ist und die Gegenflächen mindestens drei im wesentlichen kongruente, voneinander getrennte Flächen (13 -19) umfassen, die kleiner als die Hauptfläche (10) ausgebildet und von der Hauptfläche (10) teilweise überdeckt angeordnet sind, so daß bei einer Bewegung der Hauptfläche (10) parallel zu den Gegenflächen (13 - 19) die Einzelkapazitäten zwischen der Hauptfläche (10) und den Gegenflächen (13 - 19) sich ändern, wobei mindestens ein Kapazitätswert zwischen der Hauptfläche (10) und einer Gegenfläche (13 - 19) steigt und ein anderer sinkt, und daß die Kondensatorflächen (10; 13 - 19) derart mit Kapazitätsmeßorganen und Rechenschaltungen verbunden sind, daß aus den Differenzen der Einzelkapazitäten (C13 - C16) die Horizontalkräfte bestimmt werden.

2. Meßanordnung nach Anspruch 1, **dadurch gekennzeichnet,** daß der Hauptfläche (10) drei oder vier im wesentlichen kongruente, voneinander getrennte kleinere Flächen (13 bis 19) gegenüberliegend angeordnet sind, die mit ihren äußeren Rändern über den Außenrand der Hauptfläche (10) hervorragen.

3. Meßanordnung nach einem der vorhergehenden Ansprüche,

**dadurch gekennzeichnet,**
daß die Hauptfläche (10) und die Gegenflächen (13 bis 19) derart über die Kapazitätsmeßorgane und die Rechenschaltungen miteinander verschaltet sind, daß an den Ausgängen der Rechenschaltungen die Summe der Einzelkapazitäten ($C_{13} + C_{14} + C_{15} + C_{16} \triangleq K_z$) entsprechend der Vertikalkraft ($K_z$) und die Summen bzw. Differenzen der Einzelkapazitäten ($C_{13} + C_{14} - C_{15} - C_{16} \triangleq K_x$; $C_{16} + C_{13} - C_{14} - C_{15} \triangleq K_y$), entsprechend den Horizontalkräften ($K_x$, $K_y$), bezogen auf die Gesamtkapazität bzw. die Vertikalkraft ($K_x/K_z$; $K_y/K_z$) am Ausgang der Rechenschaltungen anstehen.

4. Meßanordnung nach einem der vorhergehenden Ansprüche,
**dadurch gekennzeichnet,**
daß eine Vielzahl von Hauptflächen (10) und Gegenflächen (13 bis 19) vorgesehen sind, die gleichartig ausgebildet und äquidistant mit jeweils korrespondierenden Haupt- bzw. Gegenflächen in einer Matrixanordnung angeordnet sind.

5. Meßanordnung nach Anspruch 4,
**dadurch gekennzeichnet,**
daß die Matrixausgänge über Multiplexer-/Demultiplexeranordnungen mit Meßvorrichtungen zur Bestimmung der einzelnen Kapazitätswerte verschaltet sind.

6. Meßanordnung nach einem der Ansprüche 4 oder 5,
**dadurch gekennzeichnet,**
daß die Kondensatorflächen (10 bis 19) als dünne Schichten ausgebildet und auf Kunststoffolien (30, 31) aufgebracht sind, die unter Zwischenschaltung des elastomeren Dielektrikums (20, 21) miteinander verbunden sind, wobei die Kunstoffolien (30, 31) zwischen den Kondensatorflächen (13 bis 19) durch Schnitte oder Kerben (40) durchtrennt sind.

7. Meßanordnung nach einem der vorhergehenden Ansprüche,
**dadurch gekennzeichnet,**
daß das Dielektrikum (20, 21) mindestens ein vorzugsweise geschäumtes Elastomer umfaßt.

8. Meßanordnung nach einen der vorhergehenden Ansprüche,
**dadurch gekennzeichnet,**
daß das Dielektrikum aus mindestens zwei zu den Flächen planparallelen Schichten (20, 21) besteht, die gegenläufige Temperaturkoeffizienten aufweisen und derart gestaltet sind, daß der gemeinsame Temperaturkoeffizient im wesentlichen zu Null wird.

9. Meßanordnung nach einem der vorhergehenden Ansprüche,
**dadurch gekennzeichnet,**
daß über und unter den Kondensatorflächen (13 bis 19), also außerhalb des Kondensator-Raumes, Abschirmungen unter Zwischenschaltung von Dielektrika vorgesehen sind.

**Claims**

1. Capacitive measuring arrangement for determining forces and/or pressures with capacitor surfaces (10; 13 - 19), which are arranged in two planes, interposed with a dielectric (20, 21), and lie substantially opposite one another, the capacitor surfaces (10) in the one plane relative to the capacitor surfaces (13 - 19) being held movably in the direction of flexible rear elements in the other plane, characterised in that the capacitor surfaces comprise a main surface (10) which is arranged in the one plane, whilst all the other capacitor surfaces are arranged as opposing surfaces (13 - 19) with respect to the main surface (10) in the other plane, interposed by the dielectric (20, 21), the main surface (10) being movable both perpendicularly and in parallel relative to the opposing surfaces (13 - 19) and the opposing surfaces comprising at least three substantially congruent surfaces (13 - 19), separated from one another, which are smaller than the main surface (10) and are arranged partially covered by the main surface (10) so that, in the event of a movement of the main surface (10) parallel to the opposing surfaces (13 - 19), the individual capacitances between the main surface (10) and the opposing surfaces (13 - 19) change, at least one capacitance value increasing and another decreasing between the main surface (10) and the opposing surfaces (13 - 19), and that the capacitor surfaces (10; 13 - 19) are connected to capacitance measuring elements and arithmetic circuits in such a manner that the horizontal forces are determined from the differences between the individual capacitances (C13 - C16).

2. Measuring arrangement according to claim 1, characterised in that three or four substantially congruent smaller surfaces (13 to 19), separated from one another, are arranged opposing the main surface (10), the outer edges of said smaller surfaces (13 to 19) projecting over the outer edge of the main surface (10).

3. Measuring arrangement according to one of the preceding claims, characterised in that the main surface (10) and the opposing surfaces (13 to 19) are interconnected via the capacitance measuring elements and the arithmetic circuits in such a manner that the sum of the individual capacitances ($C_{13}$ + $C_{14}$ + $C_{15}$ + $C_{16}$ ≙ $K_z$) corresponding to the vertical force ($K_z$) and the sums or differences of the individual capacitances ($C_{13}$ + $C_{14}$ - $C_{15}$ - $C_{16}$ ≙ $K_x$; $C_{16}$ + $C_{13}$ - $C_{14}$ - $C_{15}$ ≙ $K_y$) corresponding to the horizontal forces $K_x/K_y$), related to the total capacitance or the vertical force ($K_x/K_z$; $K_y/K_z$) at the output of the arithmetic circuits, are present at the outputs of the arithmetic circuits.

4. Measuring arrangement according to one of the preceding claims, characterised in that a plurality of main surfaces (10) and opposing surfaces (13 to 19), which are designed in the same way, are provided and the corresponding main and opposing surfaces are arranged equidistantly in a matrix arrangement.

5. Measuring arrangement according to claim 4, characterised in that the matrix outputs are connected via multiplexer/demultiplexer arrangements to measuring devices for determining the individual capacitance values.

6. Measuring arrangement according to one of claims 4 or 5, characterised in that the capacitor surfaces (10 to 19) are designed as thin layers and are applied to plastic films (30, 31) which are connected to one another, interposed by the elastomeric dielectric (20, 21), the plastic films (30, 31) between the capacitor surfaces (13 to 19) being cut through by cuts or notches (40).

7. Measuring arrangement according to one of the preceding claims, characterised in that the dielectric (20, 21) comprises at least one preferentially foamed elastomer.

8. Measuring arrangement according to one of the preceding claims, characterised in that the dielectric consists of at least two layers (20, 21) plane parallel with respect to the surfaces, said layers (20, 21) having opposed temperature coefficients and being designed in such a manner that the combined temperature coefficient substantially approaches zero.

9. Measuring arrangement according to one of the preceding claims, characterised in that screens, interposed with dielectrics, are provided over and under the capacitor surfaces (13 to 19), and therefore outside the capacitor space.

## Revendications

1. Dispositif de mesure capacitif pour la détermination de forces et/ou de pressions, avec des surfaces de condensateurs (10; 13 à 19), disposées dans deux plans avec un diélectrique intercalaire (20, 21) et essentiellement en vis-à-vis les unes par rapport aux autres, les surfaces de condensateurs (10), dans l'un des plans, étant maintenues par rapport aux surfaces de condensateurs (13 à 19), dans l'autre plan, avec une possibilité de déplacement contre des éléments de retour élastiques, caractérisé en ce que les surfaces de condensateurs comportent une surface principale (10), disposée dans l'un des plans, tandis que toutes les autres surfaces de condensateurs sont disposées, sous forme de contre-surfaces (13 à 19) par rapport à la surface principale (10), dans l'autre plan, avec un diélectrique (20, 21) intercalaire, la surface principale (10) présentant une possibilité de déplacement relatif, aussi bien à la perpendiculaire qu'à la parallèle des contre-surfaces (13 à 19), et les contre-surfaces comportant au moins trois surfaces (13 à 19) essentiellement congruentes, séparées les unes des autres, moins élevées que la surface principale (10) et partiellement recouvertes par cette surface (10), de sorte que les capacités individuelles entre la surface principale (10) et les contre-surfaces (13 à 19) varient lors d'un déplacement de la surface principale (10), à la parallèle des contre-surfaces (13 à 19), une valeur capacitive, au moins, s'élevant entre la surface principale (10) et une contre-surface (13 à 19), et une autre s'abaissant, et en ce que les surfaces de condensateurs (10; 13 à 19) sont reliées aux organes de mesure capacitifs et aux circuits de calcul, de sorte que les forces horizontales sont déterminées à partir des différences des capacités individuelles ($C_{13}$ à $C_{16}$).

2. Dispositif de mesure suivant la revendication 1, caractérisé en ce que trois ou quatre surfaces moins élevées (13 à 19), séparées les unes des autres et essentiellement congruentes, sont disposées en vis-à-vis de la surface principale (10), ces surfaces dépassant, par leurs bords externes, du bord externe de la surface principale (10).

3. Dispositif de mesure suivant l'une quelconque des revendications précédentes, caractérisé en ce que la surface principale (10) et les contre-surfaces (13 à 19) sont connectées entre elles, par l'intermédiaire des organes de mesure capacitifs et des circuits de calcul, de sorte que la somme des capacités individuelles ($C_{13}$ + $C_{14}$ + $C_{15}$ + $C_{16} \triangleq K_z$), correspondante à la force verticale ($K_z$), et les sommes et/ou différences des capacités individuelles ($C_{13}$ + $C_{14}$ -$C_{15}$ - $C_{16} \triangleq K_x$; $C_{16}$ + $C_{13}$ - $C_{14}$ - $C_{15} \triangleq K_y$), correspondantes aux forces horizontales ($K_x$, $K_y$), rapportées à la capacité totale et/ou à la force verticale ($K_x/K_z$; $K_y/K_z$) à la sortie des circuits de calcul, sont disponibles aux sorties de ces circuits.

4. Dispositif de mesure suivant l'une quelconque des revendications précédentes, caractérisé par une multiplicité de surfaces principales (10) et de contre-surfaces (13 à 19), d'une réalisation identique et disposées à équidistance de surfaces principales et de contre-surfaces, respectivement correspondantes, dans un agencement matriciel.

5. Dispositif de mesure suivant la revendication 4, caractérisé en ce que les sorties de la matrice sont connectées, par l'intermédiaire de montages multiplex/démultiplex, à des dispositifs de mesure pour la détermination des valeurs capacitives individuelles.

6. Dispositif de mesure suivant l'une des revendications 4 et 5, caractérisé en ce que les surfaces de condensateurs (10 à 19) sont réalisées sous forme de couches minces, et sont appliquées sur des feuilles de matière plastique (30, 31), assemblées entre elles par un diélectrique élastomère intercalaire (20, 21), les feuilles de matière plastique (30, 31) étant séparées par des entailles ou des encoches (40) entre les surfaces de condensateurs (13 à 19).

7. Dispositif de mesure suivant l'une quelconque des revendications précédentes, caractérisé en ce que le diélectrique (20, 21) comporte un élastomère au moins, expansé de préférence.

8. Dispositif de mesure suivant l'une quelconque des revendications précédentes, caractérisé en ce que le diélectrique se compose de deux couches au moins (20, 21), parallèles et planes par rapport aux surfaces, présentant des coefficients de température contraires et réalisées de sorte, que le coefficient de température commun est essentiellement réduit à zéro.

9. Dispositif de mesure suivant l'une quelconque des revendications précédentes, caractérisé en ce que des blindages, avec des diélectriques intercalaires, sont prévus au-dessus et au-dessous des surfaces de condensateurs (13 à 19), c'est-à-dire à l'extérieur de l'espace des condensateurs.

# FIG.1

# FIG.2

# FIG.3

# FIG.4

FIG.5